# EUROPÄISCHE PATENTSCHRIFT

(11) **EP 3 160 935 B1**
(45) Veröffentlichungstag und Bekanntmachung des Hinweises auf die Patenterteilung: **17.11.2021**
(21) Anmeldenummer: 15730178.9
(22) Anmeldetag: 22.06.2015
(51) Int. Cl.: C07C 209/78

(54) **VERFAHREN ZUR HERSTELLUNG VON DI- UND POLYAMINEN DERDIPHENYLMETHANREIHE**
METHOD FOR PRODUCING DI- AND POLYAMINES OF THE DIPHENYLMETHANE SERIES
PROCÉDÉ DESTINÉ À LA FABRICATION DE DI- ET POLYAMINES DE LA SÉRIE DIPHÉNYLMÉTHANE

(30) Priorität: 24.06.2014 EP 14173581
(43) Veröffentlichungstag der Anmeldung: 03.05.2017
(73) Patentinhaber: Covestro Intellectual Property GmbH & Co. KG, 51373 Leverkusen (DE)
(72) Erfinder: KNAUF, Thomas, 41542 Dormagen (DE); WERSHOFEN, Stefan, 41065 Mönchengladbach (DE); GRUNER, Klaus-Gerd, 47239 Duisburg (DE); HARTJES, Volker, 47239 Duisburg (DE)
(74) Vertreter: Levpat
(86) Internationale Anmeldenummer: PCT/EP2015/063922
(87) Internationale Veröffentlichungsnummer: WO 2015/197519

(56) Entgegenhaltungen:
- EP-A1- 0 283 757
- EP-A1- 1 616 890
- EP-A1- 2 486 975
- None

## Beschreibung

Die Erfindung betrifft ein Verfahren zur Herstellung von Di- und Polyaminen der Diphenylmethanreihe, bei dem während des Abfahrvorganges des Produktionsprozesses dafür Sorge getragen wird, dass ein Überschuss von Anilin gegenüber Formalin gewährleistet ist.

Die kontinuierliche oder teilweise diskontinuierliche Herstellung von MDA ist z. B. in EP 1 616 890 A1, US-A-5286760, EP-A-451442 und WO-A-99/40059 offenbart. Die saure Kondensation von aromatischen Aminen und Formaldehyd zu Di- und Polyaminen der Diphenylmethanreihe läuft in mehreren Reaktionsschritten ab.

Beim Aminalverfahren wird zunächst in Abwesenheit eines sauren Katalysators Formaldehyd mit Anilin zu sogenanntem Aminal kondensiert, wobei Wasser abgespalten wird. Danach erfolgt die Umlagerung zum MDA säurekatalysiert in einem ersten Schritt zu para- bzw. ortho-Aminobenzylanilin. Die Aminobenzylaniline lagern in einem zweiten Schritt zum MDA um. Hauptprodukte der säurekatalysierten Reaktion von Anilin und Formaldehyd sind das Diamin 4,4'-MDA, seine Stellungsisomere 2,4'-MDA und 2,2'-MDA sowie höhere Homologe.

Beim Neutralisationsverfahren werden in Anwesenheit eines sauren Katalysators Anilin und Formaldehyd direkt zu Aminobenzylanilinen umgesetzt, die anschließend weiter zu den zweikernigen MDA-Isomeren und höherkernigen MDA-Homologen reagieren.

Unabhängig von der Verfahrensvariante zur Herstellung des sauren Reaktionsgemisches wird dessen Aufarbeitung gemäß dem Stand der Technik durch Neutralisation mit einer Base eingeleitet. Die Neutralisation erfolgt üblicherweise bei Temperaturen von beispielsweise 90 °C bis 100 °C ohne Zusatz weiterer Substanzen. (H.J. Twitchett, Chem. Soc. Rev. 3(2), 223 (1974)). Sie kann aber auch auf einem anderen Temperaturniveau erfolgen, um z.B. den Abbau von störenden Nebenprodukten zu beschleunigen. Hydroxide der Alkali- und Erdalkalielemente sind als Basen geeignet. Vorzugsweise kommt wässrige NaOH zum Einsatz.

Im Anschluss an die Neutralisation wird in einem Trennbehälter die organische von der wässrigen Phase getrennt. Die nach Abtrennung der wässrigen Phase verbleibende, Roh-MDA enthaltende organische Phase wird weiteren Aufarbeitungsschritten unterzogen, wie z.B. einer Wäsche mit Wasser (Basenwäsche), um Restsalze aus dem Roh-MDA zu waschen. Abschließend wird das so gereinigte Roh-MDA von überschüssigem Anilin, Wasser und anderen im Gemisch vorhandenen Stoffen (z.B. weiteren Lösungsmitteln) durch geeignete Verfahren wie z.B. Destillation, Extraktion oder Kristallisation befreit. Die nach dem Stand der Technik übliche Aufarbeitung ist beispielsweise offenbart in EP 1 652 835 A1, Seite 3, Zeile 58 bis Seite 4, Zeile 13 oder EP 2 103 595 A1, Seite 7, Zeile 21 bis 37.

EP 2 486 975 A1 offenbart die Anwendung eines speziellen Mischer-Reaktors in der Herstellung von MDA. Es wird beschrieben, dass ein lokaler Formaldehydüberschuss zur Bildung netzartiger Polymere führen kann. Der Patentanmeldung sind jedoch keinerlei Details zur Ausgestaltung des Abfahrens der Reaktion, also der Unterbrechung des Verfahrens, zu entnehmen. Insbesondere lehrt die Patentanmeldung nicht, dass *während des Abfahrvorgangs* das "A/F-Verhältnis" (das molare Verhältnis von Anilin zu Formaldehyd) über dem A/F-Verhältnis *während des Regelbetriebs* liegen sollte.

EP 1 616 890 A1 lehrt, dass Anilin und Formaldehyd zunächst in Abwesenheit des sauren Katalysators zu Aminal umgesetzt werden, und das Aminal anschließend mit saurem Katalysator versetzt wird, und es bei Temperaturen von 20 °C bis 100 °C und bei Wassergehalten des so erhaltenen sauren Reaktionsgemisches von 0 bis 20 Gewichtsprozent weiter umgesetzt wird. Insbesondere wird nach der Kondensation von Formaldehyd und Anilin zum Aminal zunächst das Wasser zumindest teilweise aus dem Aminal entfernt, wobei man einen Wassergehalt von 0 bis 5 Gewichtsprozent im Aminal einstellt, und anschließend das Aminal mit saurem Katalysator versetzt, und es bei Temperaturen von 20 °C bis 100 °C und bei Wassergehalten des so erhaltenen sauren Reaktionsgemisches von 0 bis 20 Gewichtsprozent weiter umsetzt. So können Gemische der Di- und Polyamine der Diphenylmethanreihe bei Protonierungsgraden von < 15 %, bevorzugt 4 % bis 14 %, besonders bevorzugt 5 % bis 13 %, hergestellt werden. Als Protonierungsgrad wird dabei bei einprotonigen sauren Katalysatoren (wie Salzsäure) das molare Verhältnis aus der Menge an eingesetztem sauren Katalysator und der im Reaktionsgemisch vorhandenen molaren Menge an Aminfunktionen bezeichnet. Die Patentanmeldung geht in keiner Weise auf die Vorgehensweise während des Abfahrvorgangs einer großtechnischen Produktionsanlage ein. Die enthaltenen Beispiele sind Laborexperimente. Insbesondere lehrt diese Patentanmeldung nicht, dass *während des Abfahrvorgangs* das A/F-Verhältnis **über** dem A/F-Verhältnis *während des Regelbetriebs* liegen sollte.

EP 0 283 757 A1 befasst sich ebenfalls mit der Herstellung von MDA. Das beschriebene Verfahren ist gekennzeichnet durch die Zufuhr anilinfreien MDA's zu durch Kondensation von Anilin und Formaldehyd gebildeten Aminobenzylaminen vor deren durch Wärme induzierter Umlagerungsreaktion. In Beispiel 2 wird eine Verfahrensweise beschrieben, bei der ein geringer Teil des gebildeten MDA in die Umlagerungsreaktion zurückgeführt wird (vgl. auch Patentanspruch 8). Mit anderen Worten: beschrieben wird die Verschaltung einer MDA-Anlage *im kontinuierlichen Regelbetrieb.* Details zur Vorgehensweise beim Abfahren einer MDA-Anlage werden nicht beschrieben; insbesondere gibt es keine Angaben zum A/F-Verhältnis während des Abfahrens im Vergleich zum A/F-Verhältnis während der Reaktion.

WO-A-99/ 40059 lehrt, dass man zur Herstellung von Methylendi(phenylamin) durch Umsetzung von Anilin mit Formaldehyd in Gegenwart saurer Katalysatoren in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50% der gesamt einzuspeisenden Formaldehydmenge die Reaktionsmischung auf eine Temperatur von grösser 75 °C temperiert. Die Zugabe bis zu einer Menge von mindestens 50 % der gesamt einzuspeisenden Formaldehydmenge erfolgt bei einer Temperatur des Reaktionsgemisches im Kreislauf von 20 °C bis 75 °C.

Keines der vorgenannten Dokumente des Standes der Technik legt es nahe, beim Abfahren der Reaktion zur Herstellung von MDA ein A/F-Verhältnis einzusetzen, das von dem während des Regelbetriebs abweicht. So ist es im Stand der Technik durchaus üblich, im Regelbetrieb A/F-Verhältnisse einzusetzen, die über dem durch die Stöchiometrie der Reaktion vorgegebenen (2 : 1) liegen. Der Stand der Technik legt jedoch in keiner Weise nahe, während des Abfahrens *noch größere* A/F-Verhältnisse einzuhalten.

Die Güte eines Verfahrens zur Herstellung von MDA ist einerseits definiert durch den Gehalt des Produktes an unerwünschten Nebenprodukten der Reaktion. Andererseits ist die Güte eines Verfahrens dadurch definiert, dass der gesamte Prozess von Anfahren, normaler Produktion bis zum Abfahren des Prozesses ohne technischen Produktionsausfall oder Problemen, die zu einem Eingriff in den Prozess nötigen, betrieben werden kann, und dass es nicht zu Verlusten an Einsatzstoffen, Zwischenprodukten oder an Endprodukt kommt.

Solche Probleme können z.B. bei der Außerbetriebnahme (beim "Abfahren") der Aminalreaktion auftreten. Derartige Probleme können z.B. sein, dass es zur Entstehung von hochmolekularen Feststoffen kommt, die zu Anbackungen und Verblockungen an der Ausrüstung (Aminalkessel, - kühler und -abscheider und Leitungen) führen.

Den beschriebenen Verfahren des Standes der Technik gelingt es zwar, mit hoher Ausbeute MDA herzustellen, ohne dass es bei den Endprodukten zu einer Qualitätseinbuße kommt, jedoch werden nur Verfahren beschrieben, die sich im Normalbetrieb befinden. Abfahrprozesse bis zum Erreichen eines Anlagenstopps (sogenannte "Abfahrzeit") werden nicht berücksichtigt.

An- und Abfahrzeiten treten im industriellen Alltag häufig auf und sind nicht zwangsläufig mit einem Öffnen oder einem anderen mechanischen Eingriff in einen Reaktor oder einen anderen Apparat der Anlage verbunden, sondern können auch mit dem Abstellen und erneuten Starten der Produktionsanlage aus diversen anderen Gründen in Verbindung stehen, wie z.B. Rohstoffmangel. Diese Abfahrzeiten zeichnen sich in der Praxis dadurch aus, dass es zu Abweichungen im angestrebten molaren Mengenverhältnis von Anilin gegenüber Formalin kommen kann. Wünschenswert wäre daher ein verbessertes Verfahren zur Herstellung von Di- und Polyaminen der Diphenylmethanreihe, wobei das Augenmerk auf den Zeitraum des Abfahrens der Reaktion gelegt wird. Die vorliegende Erfindung hat sich die Aufgabe gestellt, ein solches Verfahren bereitzustellen.

Erfindungsgemäß gelöst wird diese Aufgabe durch ein Verfahren zur Herstellung von Di- und Polyaminen der Diphenylmethanreihe (MDA) durch Umsetzung von Anilin (1) und Formaldehyd (2) bei einem molaren Verhältnis von Anilin (1) zu Formaldehyd (2) im Regelbetrieb von A/F_{Regelbetrieb}, welches bevorzugt einen Wert von 1,5 bis 20, besonders bevorzugt von 1,5 bis 15, ganz besonders bevorzugt von 1,5 bis 10 und außerordentlich ganz besonders bevorzugt von 1,5 bis 6, aufweist, umfassend die Schritte:
Entweder gemäß einer Variante A)
   A.I) Reaktion von Anilin (1) und Formaldehyd (2) in einem Reaktor in Abwesenheit eines sauren Katalysators (3) unter Bildung eines Aminals, wobei Anilin (1) mit einem Massenstrom m₁ und Formaldehyd mit einem Massenstrom m₂ in den Reaktor eingetragen werden und anschließende Trennung des erhaltenen Reaktionsgemisches in eine wässrige und eine organische, Aminal enthaltende Phase;
   A.II) Reaktion zumindest eines Teils der in Schritt I) erhaltenen organischen, Aminal enthaltenden Phase in einem Reaktor mit Säure (3), wobei das Aminal zu Di- und Polyaminen der Diphenylmethanreihe reagiert;
   wobei die Schritte A.I) und A.II) kontinuierlich durchgeführt werden und wobei zum Beenden der Herstellung der Di- und Polyamine der Diphenlymethanreihe die folgenden Schritte durchlaufen werden:
   A.I.1) Reduktion des Massenstroms m₂ des Formaldehyds (2) in den Reaktor aus Schritt A.I) beginnend bei einem Zeitpunkt t₀, bis m₂ zu einem Zeitpunkt t₁ null beträgt, wobei t₁ > t₀;
   A.I.2) Reduktion des Massenstroms m₁ des Anilins in den Reaktor aus Schritt A.I) bis m₁ zu einem Zeitpunkt t₂ null beträgt, wobei t₂ > t₁ und wobei die Zeitspanne t₂ ― t₁ > 0,5 Stunden bis < 30 Stunden beträgt;
   A.II.1) Reduktion des Massenstroms m₃ an Säure (3), bis m₃ null beträgt;
      und
   wobei die Reduktion der Massenströme m₁ und m₂ derart geschieht, dass bis zum Erreichen des Zeitpunkts t₁ das momentane molare Verhältnis von
   dem Reaktor aus Schritt A.I) zugegebenem Anilin (1)
      zu
   dem Reaktor aus Schritt A.I) zugegebenem Formaldehyd (2),
   A/F_{mom.}, stets ≥ 2 beträgt, wobei A/F_{mom.} in allen Ausführungsformen bis zum Erreichen des Zeitpunkts t₁ stets ≥ 1,05 · A/F_{Regelbetrieb} beträgt;
oder gemäß einer Variante B)
   B.I) Reaktion von Anilin (1) und Säure (3) in einem Reaktor unter Bildung eines Reaktionsgemisches, das das Aniliniumsalz der verwendeten Säure (3) enthält;
   B.II) Reaktion zumindest eines Teils des in Schritt B.I) erhaltenen Reaktionsgemisches mit Formaldehyd (2) in einem Reaktor, optional unter Zufuhr weiteren Anilins (1), optional unter Zufuhr weiterer Säure (3), wobei Di- und Polyamine der Diphenylmethanreihe entstehen;
   wobei die Schritte B.I) und B.II) kontinuierlich durchgeführt werden und wobei zum Beenden der Herstellung der Di- und Polyamine der Diphenlymethanreihe die folgenden Schritte durchlaufen werden:
   B.II.1) Reduktion des Massenstroms m₂ des Formaldehyds (2) in den Reaktor aus Schritt B.II) beginnend bei einem Zeitpunkt t₀, bis m₂ zu einem Zeitpunkt t₁ null beträgt, wobei t₁ > t₀;
   B.I.1) Reduktion des Massenstroms m₁ des Anilins in den Reaktor aus Schritt B.I) sowie ggf. in den Reaktor aus Schritt B.II), bis m₁ zu einem Zeitpunkt t₂ null beträgt, wobei t₂ > t₁ und wobei die Zeitspanne t₂ ― t₁ > 0,5 Stunden bis < 30 Stunden beträgt;
   B.I.2) Reduktion des Massenstroms m₃ der Säure (3) in den Reaktor aus Schritt B.I) sowie ggf. in den Reaktor aus Schritt B.II), bis m₃ null beträgt;
      und
   wobei die Reduktion der Massenströme m₁ und m₂ derart geschieht, dass bis zum Erreichen des Zeitpunkts t₁ das momentane molare Verhältnis von
   dem Reaktor aus Schritt B.I) zugegebenem Anilin (1) und, sofern vorhanden, dem Reaktor aus Schritt B.II) zugegebenem Anilin (1)
      zu
   dem Reaktor aus Schritt B.II) zugegebenem Formaldehyd (2),
   A/F_{mom.}, stets ≥ 2 beträgt, wobei A/F_{mom.} in allen Ausführungsformen bis zum Erreichen des Zeitpunkts t₁ stets ≥ 1,05 · A/F_{Regelbetrieb} beträgt.

Der Abfahrvorgang kann dabei aus einem Zustand der Voll-Last, Teil-Last oder für die betreffende Anlage spezifischen Minimal-Last heraus erfolgen. Unabhängig von der vorliegenden Last wird im Rahmen der vorliegenden Erfindung der Zustand, aus dem heraus der Abfahrvorgang zum Zeitpunkt t₀ eingeleitet wird, als *Regelbetrieb* bezeichnet.

Im Sinne der vorliegenden Erfindung werden unter *Di- und Polyaminen der Diphenylmethanreihe* Amine und Gemische von Aminen folgenden Typs verstanden:

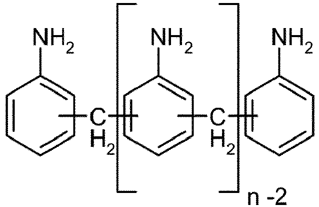

Dabei steht n für eine natürliche Zahl ≥ 2. Im Folgenden werden die Verbindungen dieses Typs, bei denen n = 2 ist, auch als Diamine der Diphenylmethanreihe oder Diaminodiphenylmethane (nachfolgend MMDA) bezeichnet. Verbindungen dieses Typs, bei denen n > 2 ist, werden im Rahmen dieser Erfindung auch als Polyamine der Diphenylmethanreihe oder Polyphenylenpolymethylenpolyamine (nachfolgend PMDA) bezeichnet. Gemische aus beiden Typen werden auch als Di- und Polyamine der Diphenylmethanreihe bezeichnet (nachfolgend MDA). Industriell werden die Di- und Polyamingemische überwiegend durch Phosgenierung zu den entsprechenden Di- und Polyisocyanaten der Diphenylmethanreihe umgesetzt.

In beiden Varianten können die *Reaktoren aus den Schritten I) bzw. II)* gleich oder verschieden sein. Das heißt, in Variante A) ist es sowohl möglich, das in Schritt A.I) gebildete Aminal im Reaktor zu belassen und die Säure zuzugeben, als auch das Aminal in einen anderen Reaktor zu überführen und dort dann die Säure (3) zuzugeben. In Variante B) ist es sowohl möglich, das in Schritt B.I) gebildete Reaktionsprodukt aus Anilin (1) und Säure (3) im Reaktor zu belassen und den Formaldehyd (2) zuzugeben, als auch das Reaktionsprodukt aus Anilin (1) und Säure (3) in einen anderen Reaktor zu überführen und dort dann den Formaldehyd (2) zuzugeben. Des Weiteren umfasst der Begriff "*ein Reaktor"* im Rahmen der vorliegenden Erfindung auch den Fall, dass eine Reaktorkaskade eingesetzt wird (mit anderen Worten, das Wort "*ein*" ist in diesem Zusammenhang als unbestimmter Artikel und nicht als Zahlwort aufzufassen).

In beiden Varianten werden die Schritte I) und II) kontinuierlich durchgeführt.

Das *momentane molare Verhältnis, A*/*F*_{*mom*.}, im Zeitraum bis t₁ ergibt sich **im Fall der Variante A)** in einfacher Weise aus den bekannten Einsatzströmen (1) und (2) in den Reaktor aus Schritt A.I) zu einem bestimmten Zeitpunkt t. **Im Fall der Variante B)** ergibt sich das momentane molare Verhältnis, A/F_{mom.}, im Zeitraum bis t₁ in analoger Weise aus den bekannten Einsatzströmen (1) und (2) in den Reaktor aus Schritt B.I) bzw. in den Reaktor aus Schritt B.II) zu einem bestimmten Zeitpunkt t. Wird von der Möglichkeit Gebrauch gemacht, in Schritt B.II) weiteres Anilin in den Reaktor aus Schritt B.II) einzutragen, so wird dieses zum Zwecke der Bestimmung des momentanen molaren Verhältnisses, A/F_{mom.}, im Zeitraum bis t₁ zum Anilin aus Schritt B.I) addiert. Wird solches in Schritt B.II) zugesetztes Anilin zuvor mit Säure vermischt, sodass es als Aniliniumsalz vorliegt, so ändert dies nichts an der Berechnung, da ein Mol Anilin mit einem Mol Säure zu einem Mol Aniliniumsalz reagiert. Für die Zwecke der Berechnung des momentanen molaren Verhältnisses A/F_{mom.} kann also so getan werden, als läge alles Anilin (1) in freier Form vor.

Erfindungsgemäß ist vorgesehen, dass die Reduktion der Massenströme m₁ und m₂ (beispielsweise ausgedrückt in kg Einsatzanilin/Stunde und kg Formaldehyd/Stunde oder kg FormaldehydLösung/Stunde) derart geschieht, dass bis zum Erreichen des Zeitpunkts t₁ A/F_{mom.}, stets ≥ 2 beträgt, wobei A/F_{mom.} in allen Ausführungsformen bis zum Erreichen des Zeitpunkts t₁ ≥ 1,05 · A/F_{Regelbetrieb} beträgt. Dieses gilt aus denkgesetzlichen Gründen nicht zum Zeitpunkt t₁ selbst, da dann der Massenstrom m₂ des Formaldehyds null beträgt. Auf jeden Fall ist es aber vorgesehen, dass die Verringerung des Formaldehyd-Eintrags so erfolgt, dass während des Abfahrvorgangs im jeweiligen Reaktor mindestens die für die Bildung von MDA erforderliche stöchiometrische Menge an Anilin vorhanden ist.

In beiden Varianten erfolgt im **ersten Schritt** des Abfahrvorganges (Schritt A.I.1) bzw. B.II.1)) im Zeitraum von t₀ bis t₁ die vorzugsweise stufenlose Drosselung der Formaldehydzufuhr in den jeweiligen Reaktor auf null, während die Anilindosierung mit gleicher, größerer oder mit verminderter Last weiterläuft. Dieses ermöglicht das Abreagieren des im Reaktor aus Schritt A.I) bzw. B.II) befindlichen Formaldehyds und das Ausschleusen des entstandenen Produktes aus diesem Reaktor, bis in Variante A im Aminalreaktor und in den Apparaten der weiteren Prozessschritte praktisch nur noch Anilin enthalten ist, beziehungsweise in Variante B im Reaktor des Schrittes B.II) und in den Apparaten der weiteren Prozessschritte praktisch nur noch Anilin enthalten ist.

Im **zweiten Schritt** des Abfahrvorganges (Schritt A.I.1) bzw. B.I.1)) erfolgt schließlich das Abstellen der Anilinzufuhr.

Im **dritten Schritt** des Abfahrvorgangs (Schritt A.II.1) bzw. B.I.2)) erfolgt das Abstellen der Säurezufuhr. In beiden Varianten ist es bevorzugt, den Massenstrom m₃ an Säure (3) frühestens beginnend beim Zeitpunkt t₁ zu reduzieren. Besonders bevorzugt ist es in beiden Varianten, den vor Beginn des Abfahrprozesses (d. h. vor Durchführung der Schritte A.I.1) bzw. B.II.1)) herrschenden Massenstrom m₃ bis zum Zeitpunkt t₂ beizubehalten und dann die Zufuhr weiterer Säure (3) einzustellen (etwa durch sofortiges Schließen eines Ventils), sodass allenfalls noch in den Zufuhrleitungen befindliche Restmengen an Säure (3) für eine gewisse Zeit weiter zugeführt werden. Auf diese Weise wird sichergestellt, dass in der sich anschließenden Aufarbeitung (Neutralisation) hinreichend große Salzmengen vorhanden sind, was die Phasentrennung erleichtert.

Ausführungsformen des erfindungsgemäßen Verfahrens werden nachfolgend beschrieben. Sie können beliebig miteinander kombiniert werden, sofern sich aus dem Kontext nicht eindeutig das Gegenteil ergibt.

Während des Abfahrprocederes liegt im Fall der Variante A) die Reaktionstemperatur im Reaktorraum des Reaktors aus Schritt A.I) beispielsweise bei 20 °C bis 120 °C, bevorzugt 40 °C bis 110 °C und ganz bevorzugt 60 °C bis 100 °C. Der Reaktor aus Schritt A.I) (Aminalreaktor) wird bei Normaldruck oder im Überdruck betrieben. Bevorzugt liegt ein Druck von 1,05 bis 5 bar absolut, ganz bevorzugt von 1,1 bis 3 bar und ganz besonders bevorzugt von 1,2 bar bis 2 bar absolut vor. Die Druckhaltung erfolgt durch Druckregelungsventile, oder indem man die Abgassysteme von Aminalreaktor und dem Überlauf des Aminalabscheiders verbindet. Der Aminalabscheider und der Ablauf der wässrigen Phase sind vorzugsweise beheizt, um Anbackungen zu verhindern.

Während des Abfahrprocederes liegt im Fall der Variante B) die Reaktionstemperatur im Reaktorraum des Reaktors aus Schritt B.II) beispielsweise bei 20 °C bis 200 °C und bevorzugt 20 °C bis 160 °C. Vor dem Abfahren liegt die Temperatur bei > 75 °C, während des Abfahrens sinkt die Temperatur, wenn der Reaktor nicht beheizt wird.

Nach dem Abfahren können die Apparate mit darin noch befindlichem Anilin stehengelassen werden. Alternativ können einer, mehrere oder alle Apparate entleert werden, um sie z.B. auf Instandhaltungsmaßnahmen vorzubereiten.

Erfindungsgemäß ist vorgesehen, dass in Schritten I.1) und I.2) die Reduktion der Massenströme m₁ und m₂ (beispielsweise ausgedrückt in kg Einsatzanilin/Stunde und kg Formaldehyd/Stunde oder kg Formaldehyd-Lösung/Stunde) derart geschieht, dass bis zum Erreichen des Zeitpunkts t₁ das molare Verhältnis von Anilin zu Formaldehyd im ersten Reaktor ≥ 2:1 beträgt. Dieses gilt aus denkgesetzlichen Gründen nicht zum Zeitpunkt t₁ selbst, da dann der Massenstrom m₂ des Formaldehyds null beträgt. Auf jeden Fall ist es aber vorgesehen, dass die Verringerung des Formaldehyd-Eintrags so erfolgt, dass im Reaktor mindestens die für die Bildung von MDA erforderliche stöchiometrische Menge an Anilin vorhanden ist.

Sollten zwei oder mehr MDA-Reaktorlinien parallel betrieben werden, dann kann, muss aber nicht, zunächst eine Reaktorlinie abgefahren und die anderen Reaktorlinien nacheinander abgefahren werden. Es ist aber auch möglich alle MDA-Reaktorlinien zeitnah abzufahren.

In einer weiteren Ausführungsform des erfindungsgemäßen Verfahrens wird weiterhin während und/oder nach Schritt A.I) bzw. B.II) das erhaltene Gemisch aus dem Reaktor ausgetragen. Im Ergebnis kann so der Produktionsprozess einschließlich der beteiligten Stofftransporte weitergeführt werden. Vorteilhafterweise wird das Austragen so lange fortgesetzt, bis in Variante A im Aminalreaktor und in den Apparaten der weiteren Prozessschritte praktisch nur noch Anilin enthalten ist, beziehungsweise in Variante B im Reaktor des Schrittes B.II) und in den Apparaten der weiteren Prozessschritte praktisch nur noch Anilin enthalten ist.

In einer weiteren Ausführungsform des erfindungsgemäßen Verfahrens beträgt in beiden Varianten A) und B) t₂ ― t₁ > 0,5 Stunden bis < 30 Stunden. Vorzugsweise liegt diese Zeitspanne bei > 0,5 Stunden bis < 10 Stunden und besonders bevorzugt bei > 1 Stunde bis < 5 Stunden.

In einer weiteren Ausführungsform des erfindungsgemäßen Verfahrens beträgt in beiden Varianten A) und B) in Schritt I) der Massenstrom m₁ ≥ 1000 kg/Stunde. Vorzugsweise beträgt dieser Massenstrom ≥ 2000 kg/Stunde bis ≤ 200000 kg/Stunde, mehr bevorzugt bei ≥ 3000 kg/Stunde bis ≤ 100000 kg/Stunde.

In einer weiteren Ausführungsform des erfindungsgemäßen Verfahrens beträgt in beiden Varianten A) und B) in Schritt I) bzw. in Schritt II) der Massenstrom m₂ ≥ 300 kg/Stunde. Vorzugsweise beträgt dieser Massenstrom ≥ 400 kg/Stunde bis ≤ 100000 kg/Stunde, mehr bevorzugt bei ≥ 500 kg/Stunde bis ≤ 50000 kg/Stunde.

Der eingesetzte Formaldehyd (2) kann in beiden Varianten aus allen bekannten Produktionsverfahren für Formaldehyd stammen. Lediglich beispielhaft sei das Silberkontakt-Verfahren erwähnt.

In einer weiteren Ausführungsform des erfindungsgemäßen Verfahrens liegt in beiden Varianten A) und B) nach dem Zeitpunkt t₂ zumindest der Reaktor aus Schritt A.I) bzw. aus Schritt B.I) zumindest teilweise mit Anilin befüllt vor; d.h. nach Beendigung der Anilinzufuhr im jeweiligen Reaktor noch befindliches Anilin (oder Aniliniumsalz) wird nicht vollständig ausgetragen. Beim Verbleib von Anilin (oder Aniliniumsalz) im jeweiligen Reaktor ohne die Gegenwart von Formaldehyd kann eine Unterbrechung der Produktion ohne die Bildung unerwünschter hochmolekularer Nebenprodukte erfolgen.

Die Verfahrensführungen der Varianten A) und B) im Normalbetrieb bis zum Erhalt des Rohprodukts werden im Folgenden näher beschrieben:
Die Herstellung von Di- und/oder Polyaminen der Diphenylmethanreihe nach Variante A) lässt sich exemplarisch wie folgt zusammenfassen:
a) Kernvorgang des Schritts I): Anilin und Formaldehyd werden in Abwesenheit eines sauren Katalysators zu Aminal und Wasser kondensiert, und das dabei entstehende Aminal wird aus dem Aminalreaktor ausgetragen, und
b) Wasser aus Schritt a), das im Wesentlichen aus Kondensationswasser der Aminalreaktion und Wasser aus dem Einsatzstoff Formaldehyd herrührt, wird zumindest teilweise aus dem Reaktionsgemisch der Aminalreaktion als wässrige Phase abgetrennt, und
c) Kernvorgang des Schritts II): das Aminal aus Schritt b) wird säurekatalysiert zu MDA umgelagert.

Die Kondensation von Anilin und Formaldehyd in **Schritt a)** kann nach einem Verfahren nach dem Stand der Technik durchgeführt werden. Dabei werden normalerweise Anilin und wässrige Formaldehydlösung bei Molverhältnissen im Bereich von 1,5 bis 20, bevorzugt von 1,5 bis 15, besonders bevorzugt von 1,5 bis 10 und ganz besonders bevorzugt von 1,5 bis 6 bei Temperaturen von 20 °C bis 120 °C, bevorzugt 40 °C bis 110 °C und besonders bevorzugt 60 °C bis 100 °C zu Aminal und Wasser kondensiert. Die Umsetzung erfolgt üblicherweise bei Normaldruck. Geeignete Anilinqualitäten sind z.B. beschrieben in EP 1 257 522 B1, EP 2 103 595 A1 und EP 1 813 598 B1. Es werden bevorzugt technische Qualitäten an Formalin (wässrige Lösung von Formaldehyd) mit 30 Massen-% bis 50 Massen-% Formaldehyd in Wasser eingesetzt. Jedoch sind auch Formaldehydlösungen mit niedrigeren oder höheren Konzentrationen oder auch der Einsatz gasförmigen Formaldehyds denkbar.

In **Schritt b)** erfolgt die Phasentrennung von organischer Aminalphase und wässriger Phase bei Temperaturen von 20 °C bis 120 °C, bevorzugt von 40 °C bis 110 °C, besonders bevorzugt von 60 °C bis 100 °C, bevorzugt bei Umgebungsdruck. Ferner kann die Phasentrennung auch bei leichtem Überdruck durchgeführt werden.

Die Umlagerung des Aminals in **Schritt c)** erfolgt in Gegenwart eines sauren Katalysators, üblicherweise einer starken Mineralsäure wie Salzsäure. Bevorzugt ist der Einsatz von Mineralsäure in einem molaren Verhältnis Mineralsäure zu Anilin von 0,001 bis 0,9, bevorzugt 0,05 bis 0,5. Es können natürlich auch feste, saure Katalysatoren, wie in der Literatur beschrieben, verwendet werden. Dabei kann Formaldehyd in eine Mischung von Anilin und saurem Katalysator gegeben und die Reaktionslösung durch stufenweises Erhitzen ausreagiert werden. Alternativ können auch Anilin und Formaldehyd zunächst vorreagiert werden und anschließend mit oder ohne vorhergehender Wasserabtrennung mit dem sauren Katalysator oder einem Gemisch von weiterem Anilin und saurem Katalysator versetzt werden, wonach die Reaktionslösung durch stufenweises Erhitzen ausreagiert wird. Diese Reaktion kann kontinuierlich nach einem der zahlreichen in der Literatur beschriebenen Verfahren ausgeführt werden (z. B. in EP 1 616 890 A1).

Die Herstellung der rohen Di- und/oder Polyamine der Diphenylmethanreihe nach **Variante B)** lässt sich exemplarisch wie folgt zusammenfassen:
a) Kernvorgang des Schrittes B.I): Anilin und Säure werden in Abwesenheit von Formaldehyd zu einem Reaktionsgemisch umgesetzt, das das Aniliniumsalz der verwendeten Säure enthält, und
b) Kernvorgang des Schritts B.II): das Reaktionsgemisch aus Schritt a), das das Aniliniumsalz der verwendeten Säure enthält, wird mit Formaldehyd versetzt und zu MDA umgelagert.

Die Umsetzung von Anilin und Säure, bevorzugt Salzsäure, in **Schritt a)** kann nach einem Verfahren nach dem Stand der Technik durchgeführt werden. Die weitere Beschreibung erfolgt anhand des Beispiels wässriger Salzsäure, wobei auch andere Säuren zum Einsatz kommen können. Es werden normalerweise Anilin und eine wässrige Salzsäure bei Molverhältnissen von Anilin zu Säure im Bereich von 1,6 bis 100, bevorzugt 3,3 bis 20 umgesetzt. Diese Umsetzung kann in einem vorgelagerten Reaktor oder einer Mischstrecke erfolgen, wobei das Reaktionsgemisch fakultativ in einem Vorlagebehälter zwischengelagert werden kann. Optional kann diese Reaktion im gleichen Reaktor erfolgen, in dem auch die anschließende Umsetzung des Reaktionsgemisches von Anilin und Säure mit Formaldehyd stattfindet. Geeignete Anilinqualitäten sind z.B. beschrieben in EP 1 257 522 B1, EP 2 103 595 A1 und EP 1 813 598 B1. Geeignete Salzsäurequalitäten sind z.B. in EP 1 652 835 A1 beschrieben.

Zunächst kann "Einsatzanilin" im Reaktor bei Temperaturen von 10 °C bis 60 °C vorgelegt werden. Das Einsatzanilin setzt sich aus frischem Anilin und gegebenenfalls Anilin aus der MDA-Destillation (weiter unten näher beschrieben; siehe Schritt h)) und gegebenenfalls Anilin aus der Abwasseraufbereitung zusammen.

Dann erfolgt beispielsweise bei schon laufender Anilindosierung die Zugabe der Salzsäure in das vorgelegte Anilin, wobei für eine gute Durchmischung Sorge getragen wird. Diese gute Durchmischung kann durch Rühren mit einem Rührer erreicht werden oder auch durch ein im Kreis fahren (mittels Pumpen) der Reaktionsmischung oder durch eine Kombination aus Rühren und im Kreis fahren. Gegebenenfalls sollte die gesamte Anlage von Eduktströmen bis Produktabnahme betriebsbereit sein. Der Reaktionsapparat kann erforderlichenfalls mit einem internen oder externen Wärmeaustauscher ausgerüstet sein, um die entstehende Reaktionswärme abführen zu können. Alternativ kann auch das Einsatzanilin und/oder die Salzsäure entsprechend abgekühlt werden. Eine weitere Alternative bietet die Anwendung einer Siedekühlung zur Abführung der Reaktionswärme.

In **Schritt b)** erfolgt die Umsetzung des Anilinhydrochlorid-haltigen Reaktionsgemisches aus Schritt a) mit wässriger Formaldehydlösung. Dabei kann Formaldehyd in eine Mischung von Anilin und saurem Katalysator gegeben und die Reaktionslösung durch stufenweises Erhitzen ausreagiert werden, wie z. B. in EP 1 053 222 A1 beschrieben. Üblicherweise erfolgt die Umsetzung bei Temperaturen von 20 °C bis 150 °C.

Zweckmäßigerweise sind der Reaktor aus Schritt B.I) und der Reaktor aus Schritt B.II) voneinander verschieden. Es ist aber nicht ausgeschlossen, dass die Schritte B.I) und B.II) in demselben Reaktor ausgeführt werden. Diese Reaktion wird kontinuierlich ausgeführt.

Es werden bevorzugt technische Qualitäten an Formalin (wässrige Lösung von Formaldehyd) mit 30 Massen % bis 50 Massen % Formaldehyd in Wasser eingesetzt. Jedoch sind auch Formaldehydlösungen mit niedrigeren oder höheren Konzentrationen oder auch der Einsatz gasförmigen Formaldehyds denkbar.

Ab dem Zeitpunkt t₀ und bis zum Erreichen des Zeitpunktes t₁ wird Formaldehyd in solchen Mengen zudosiert, dass mindestens das 1,05-fache des in der Rezeptur des Regelbetriebes vorgesehenen A/F_{Regelbetrieb}-Verhältnisses eingehalten wird.

In **beiden Varianten A) und B)** wird ein rohes Reaktionsgemisch enthaltend Di- und Polyamine der Diphenylmethanreihe erhalten (in Variante A) in Schritt c) und in Variante B) in Schritt b)). Die Aufarbeitung dieses Reaktionsgemisches erfolgt unabhängig von der eingesetzten Variante A) oder B) bevorzug wie folgt:
d) Das Reaktionsgemisch enthaltend Di- und Polyamine der Diphenylmethanreihe wird neutralisiert, und
e) das neutralisierte Reaktionsgemisch enthaltend Di- und Polyamine der Diphenylmethanreihe wird in einem Trennbehälter in eine organische Phase enthaltend Di- und Polyamine der Diphenylmethanreihe und eine wässrige Phase aufgetrennt, und
f) die organische Phase enthaltend Di- und Polyamine der Diphenylmethanreihe wird in einem Waschbehälter mit Waschflüssigkeit weiter gereinigt, und
e) das neutralisierte Reaktionsgemisch enthaltend Di- und Polyamine der Diphenylmethanreihe wird in einem Trennbehälter in eine organische Phase enthaltend Di- und Polyamine der Diphenylmethanreihe und eine wässrige Phase aufgetrennt, und
f) die organische Phase enthaltend Di- und Polyamine der Diphenylmethanreihe wird in einem Waschbehälter mit Waschflüssigkeit weiter gereinigt, und
g) das so erhaltene Gemisch wird in einem Trennbehälter in eine organische Phase enthaltend Di- und Polyamine der Diphenylmethanreihe und eine wässrige Phase aufgetrennt, und
h) die gewaschene Di- und Polyamine der Diphenylmethanreihe enthaltende organische Phase wird destillativ von Wasser und Anilin befreit.

In **Schritt d)** wird das Reaktionsgemisch enthaltend die Di- und Polyamine der Diphenylmethanreihe ggf. unter Zusatz von Wasser und / oder Anilin neutralisiert. Nach dem Stand der Technik erfolgt die Neutralisation üblicherweise bei Temperaturen von beispielsweise 90 °C bis 100 °C ohne Zusatz weiterer Substanzen. Sie kann aber auch auf einem anderen Temperaturniveau erfolgen, um z.B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung. Die zur Neutralisation eingesetzte Base wird bevorzugt in Mengen von größer als 100 %, besonders bevorzugt 105 % bis 120 % der für die Neutralisation des eingesetzten sauren Katalysators stöchiometrisch benötigten Menge eingesetzt (siehe EP 1 652 835 A1).

Anschließend wird in **Schritt e)** das neutralisierte Reaktionsgemisch enthaltend die Di- und Polyamine der Diphenylmethanreihe in eine organische Phase enthaltend Di- und Polyamine der Diphenylmethanreihe und eine wässrige Phase aufgetrennt. Dies kann durch die Zugabe von Anilin und/oder Wasser unterstützt werden. Wird die Phasentrennung durch Zugabe von Anilin und/oder Wasser unterstützt, so erfolgt deren Zugabe bevorzugt bereits unter intensivem Mischen in der Neutralisation. Dabei kann die Vermischung in Mischstrecken mit statischen Mischern, in Rührkesseln oder Rührkesselkaskaden oder aber in einer Kombination von Mischstrecken und Rührkessel erfolgen. Das neutralisierte und durch Zugabe von Anilin und/oder Wasser verdünnte Reaktionsgemisch wird anschließend bevorzugt einem Apparat zugeführt, der aufgrund seiner Konfiguration und/oder Einbauten besonders zur Auftrennung in eine organische Phase enthaltend MDA und eine wässrige Phase geeignet ist, bevorzugt Phasentrenn- oder Extraktionsvorrichtungen entsprechend dem Stand der Technik, wie sie beispielsweise in Mass-Transfer Operations, 3rd Edition, 1980, McGraw-Hill Book Co, S. 477 bis 541, oder Ullmann's Encyclopedia of Industrial Chemistry (Vol. 21, Liquid-Liquid Extraction, E. Müller et al., Seite 272-274, 2012 Wiley-VCH Verlag GmbH & Co. KGaA, Weinheim, DOI: 10.1002/14356007.b03_06.pub2) oder in Kirk-Othmer Encyclopedia of Chemical Technology (siehe "http://onlinelibrary.wiley.com/book/10.1002/0471238961", Published Online: 15 Juni 2007, Seite 22-23) beschrieben sind (Mixer-Settler-Kaskade oder Absetzbehälter).

In **Schritt f)** schließt sich eine Wäsche der organischen Phase mit Wasser und in Schritt g) eine neuerliche Abscheidung der Wasserphase zur Entfernung von Restgehalten an Salz (bevorzugt wie in DE-A-2549890, Seite 3 beschrieben) an.

In **Schritt h)** wird aus der in Schritt g) erhaltenen organischen Phase enthaltend Di- und Polyamine der Diphenylmethanreihe destillativ Wasser und Anilin abgetrennt, wie in EP 1 813 597 B1 beschrieben. Die in Schritt g) erhaltene organische Phase hat bevorzugt eine Zusammensetzung, bezogen auf das Gewicht des Gemisches, von 5-15 Gewichtsprozent Wasser, und, je nach Einsatzverhältnissen von Anilin und Formaldehyd, 5-90 Gewichtsprozent, bevorzugt 5 - 40 Gewichtsprozent Anilin und 5-90 Gewichtsprozent, bevorzugt 50 - 90 Gewichtsprozent Di- und Polyamine der Diphenylmethanreihe. Nach Austritt aus der Phasentrennung in Schritt g) hat die organische Phase enthaltend Di- und Polyamine der Diphenylmethanreihe üblicherweise eine Temperatur von 80 °C ― 150 °C.

Die so erhaltenen Di- und Polyamine der Diphenylmethanreihe können nach den bekannten Methoden unter inerten Bedingungen mit Phosgen in einem organischen Lösungsmittel zu den entsprechenden Di- und Polyisocyanaten der Diphenylmethanreihe, dem MDI, umgesetzt werden. Dabei kann die Phosgenierung nach einem der aus dem Stand der Technik bekannten Verfahren durchgeführt werden (z.B. DE-A-844896 oder DE-A-19817691).

Werden die genannten Bedingungen während des Abfahrens eingehalten, ergeben sich folgende Vorteile:
i) Vermeidung von Verblockungen und Ablagerungen im Aminalkessel, -kühler, -abscheider und der Aminal-Kühlkreislaufpumpe und somit eine Vermeidung eines zweiten Anfahrvorganges, weil die Anlage zwecks Reinigung des Equipments nicht wieder abgefahren werden und geöffnet werden muss.
ii) Einsparung von Energie, weil wegen Entstehungen von Verblockungen und Ablagerungen und der daraus resultierenden Abstellung der Anlage zwecks Reinigung des Equipments der Anfahrvorgang nicht ein zweites Mal ausgeführt werden muss.
iii) Erhöhung der Produktivität der Anlage, weil wegen Wegfall der Reinigungszeiten zur Entfernung von Verblockungen und Ablagerungen sich die Reaktorlaufzeiten erhöhen.
iv) Vermeidung bzw. Verringerung von Niederschlägen, Anbackungen und Verstopfungen am Equipment (Aminalkessel, -kühler, -abscheider und der Aminal-Kühlkreislaufpumpe) und damit einhergehend eine Verlängerung der Standzeit des Verfahrens.
v) Geringerer Abfall nach Reinigung des Equipments (hochmolekulare Feststoffe) und Einsparung von Verbrennungskosten.
vi) Vermeidung von nicht spezifikationsgerechter Ware, die durch mehrfaches schlechtes An-und Abfahren entstehen kann: solch eine qualitativ schlechte Abfahrware muss somit nicht mit qualitativ gutem MDA verschnitten werden oder sogar im schlimmsten Fall verbrannt werden.
vii) Eine bessere Phasentrennung zwischen wässriger und organischer Phase bedingt durch den Wegfall von die Phasentrennung negativ beeinflussenden hochmolekularen Verbindungen.

Die vorliegende Erfindung wird anhand der nachfolgenden Zeichnungen und Beispiele eingehend erläutert, ohne jedoch darauf beschränkt zu sein.

Es zeigen:

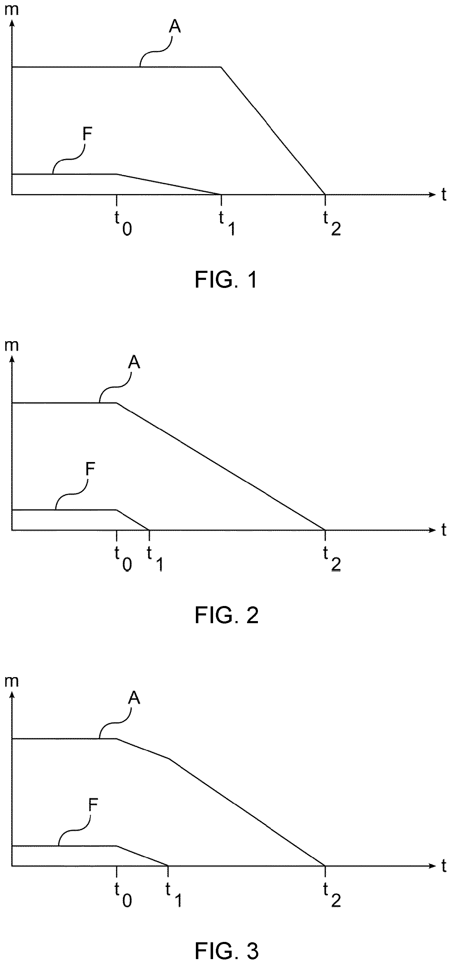

- FIG. 1-4: den zeitlichen Verlauf der Massenströme von Anilin und Formaldehyd im erfindungsgemäßen Verfahren gemäß Variante A)

FIG. 1 zeigt den zeitlichen Verlauf der Massenströme von Anilin und Formaldehyd in einer in einer Ausführungsform gemäß Variante A) des erfindungsgemäßen Verfahrens. Auf der x-Achse ist die Zeit t und auf der Y-Achse sind Massenströme m aufgetragen. Bis zum Erreichen des Zeitpunkts t₀ sind die Massenströme für Anilin (m₁, in der Figur mit "A" bezeichnet) und für Formaldehyd (m₂, in der Figur mit "F" bezeichnet) in den in der Terminologie der vorliegenden Erfindung als Reaktor aus Schritt A.I) bezeichneten Aminal-Reaktor konstant. Nun wird beschlossen, dass die Produktion heruntergefahren werden soll. Hierzu wird bei unveränderter Höhe des Massenstroms an Anilin der Massenstrom an Formaldehyd in den Aminal-Reaktor reduziert, bis er zum Zeitpunkt t₁ null beträgt.

Zum Zeitpunkt t₁ wird begonnen, den Massenstrom des Anilins in den Aminal-Reaktor zu reduzieren, bis er zum Zeitpunkt t₂ ebenfalls null beträgt. Man erkennt, dass zu jedem Zeitpunkt des Abfahrens der Reaktion der in den Aminal-Reaktor eingetragene Massenstrom des Anilins mindestens um so viel größer als der Massenstrom des Formaldehyds war, dass das molare Verhältnis von Anilin zu Formaldehyd mindestens 2 beträgt.

In der Zeit zwischen t₁ und t₂ kann das im Aminal-Reaktor noch befindliche Formaldehyd mit dem Anilin abreagieren. Letztendlich kann erreicht werden, dass im Aminal-Reaktor kein freies Formaldehyd mehr vorliegt.

Es ist selbstverständlich möglich, dass in dieser Variante nach den Zeitpunkten t₀, t₁ und/oder t₂ das erhaltene Reaktionsgemisch weiterhin aus dem Aminal-Reaktor ausgetragen wird.

FIG. 2 zeigt analog zu FIG. 1 den zeitlichen Verlauf der Massenströme von Anilin und Formaldehyd in einer weiteren Ausführungsform gemäß Variante A) des erfindungsgemäßen Verfahrens. Hier wird bereits zum Zeitpunkt t₀ auch der Massenstrom des Anilins reduziert.

FIG. 3 zeigt analog zu FIG. 2 den zeitlichen Verlauf der Massenströme von Anilin und Formaldehyd, wobei der Eintrag des Anilins in den Aminal-Reaktor in zwei verschiedenen Geschwindigkeiten reduziert wird.

### Beispiele:

### Allgemeine Bedingungen für die Herstellung von MDA bei eingefahrener Produktionsanlage (vor Beginn des Abfahrvorganges)

In einem kontinuierlichen Reaktionsprozess (Schritt a)) wurden 24,3 t/h Einsatzanilin (enthaltend 90 Massen-% Anilin) und 9,9 t/h 32 %ige wässrige Formaldehydlösung (molares Verhältnis Anilin zu Formalin 2,1 zu 1 vermischt und bei 90 °C und 1,4 bar absolut in einem gerührten Reaktionskessel zum Aminal umgesetzt. Der Reaktionskessel war mit einem Kühler mit Kühlkreislaufpumpe versehen. Das den Reaktionskessel verlassende Reaktionsgemisch wurde in einen Phasentrennapparat (Aminalabscheider) geführt (Schritt b)).

Nach der Phasentrennung zur Entfernung der wässrigen Phase wurde die organische Phase in einer Mischdüse mit 30 %iger wässriger Salzsäure versetzt (Protonierungsgrad 10 %, d. h., pro Mol Aminogruppen werden 0,1 Mol HCl zugegeben) und in den ersten Umlagerungsreaktor gefahren. Die Umlagerungsreaktion wurde in einer Reaktorkaskade bei 45 °C bis 165 °C durchgeführt (Schritt c)).

Nach vollständiger Reaktion wurde das erhaltene Reaktionsgemisch mit 32 %iger Natronlauge im molaren Verhältnis von 1,1 : 1 Natronlauge zu HCl versetzt und in einem Neutralisationsrührbehälter umgesetzt (Schritt d)). Die Temperatur betrug 115 °C. Der absolute Druck betrug 1,4 bar. Die neutralisierte Reaktionsmischung wurde anschließend in einem Neutralisationsabscheider in eine wässrige, untere Phase, die zu einem Abwassersammelbehälter geführt wird, und in eine organische Phase getrennt (Schritt e)).

Die organische, obere Phase wurde zur Wäsche geleitet und in einem gerührten Waschbehälter mit Kondensat gewaschen (Schritt f)). Nach Abtrennung des Waschwassers in einem Waschwasserabscheider (Schritt g)) wurde das so erhaltene Roh-MDA durch Destillation von Wasser und Anilin befreit, wobei als Sumpfprodukt 17 t/h MDA anfielen (Schritt h)).

### Beispiel 1 (Vergleichsbeispiel): Abfahren der MDA-Anlage, wobei zuerst Anilin abgestellt wird

Die MDA-Anlage wurde, wie in den allgemeinen Bedingungen für die Herstellung von MDA beschrieben, bei einer Last von 17 t/h MDA betrieben. Nach einem technischen Defekt in den Umlagerungsreaktoren wurde im Aminalreaktor Anilin abgestellt und Formalin 2 Minuten später abgestellt. Die Zugabe von Salzsäure wurde zum gleichen Zeitpunkt wie die Anilinzugabe unterbrochen. Der Aminalreaktor und die Umlagerungsreaktoren wurden entleert. Nach Behebung des Defekts in den Umlagerungsreaktoren wurde der leere Aminalreaktor mit Einsatzanilin (enthaltend 90 Massen-% Anilin) befüllt, bis Anilin über das Siphon in den Aminalabscheider überlief. Wenn der Aminalreaktor so gefüllt war, lief das Einsatzanilin mit einer Last von 12,2 t/h in den gerührten Aminalreaktionskessel, was 50 % der Nennlast entsprach.

Nun wurde der Formalinweg freigegeben. Die Reaktion sprang sofort an und das Reaktionsgemisch wurde auf 90 °C eingeregelt. Der Druck im Aminalreaktor lag während der Anfahrphase bei 1,4 bar absolut. Die Menge an 32 %iger wässriger Formaldehydlösung, die während einer geplanten Anfahrzeit t von 45 Minuten in den Aminalreaktor eingeleitet werden sollte, sollte stufenlos von 0 t/h auf 4,95 t/h hochgezogen werden, was 50 % der Nennlast entsprach. Nach 30 Minuten musste die Anlage allerdings abgestellt werden, weil der Aminalkessel, der Aminalkühler und der Aminalabscheider blockiert waren und die Aminal-Kühlkreislaufpumpe ebenfalls mit Feststoff geblockt war und stehenblieb.

### Beispiel 2 (Vergleichsbeispiel): Abfahren der MDA-Anlage, wobei Formalin- und Anilinzufuhr zeitnah abgefahren wurden.

Die MDA-Anlage wurde, wie in den allgemeinen Bedingungen für die Herstellung von MDA beschrieben, bei einer Last von 17 t/h MDA betrieben. Nach einem technischen Defekt in dem Aminalreaktor wurden im Wesentlichen gleichzeitig Anilin und Formalin abgestellt. Die Zugabe von Salzsäure wurde zum gleichen Zeitpunkt wie die Anilinzugabe unterbrochen.

Der Aminalreaktor wurde entleert. Nach Behebung des Defekts wurde der leere Aminalreaktor mit Einsatzanilin (enthaltend 90 Massen-% Anilin) befüllt bis Anilin über das Siphon in den Aminalabscheider überlief. Wenn der Aminalreaktor so gefüllt war, lief das Einsatzanilin mit einer Last von 12,2 t/h in den gerührten Aminalreaktionskessel, was 50 % der Nennlast entsprach.

Nun wurde der Formalinweg freigegeben. Die Reaktion sprang sofort an und das Reaktionsgemisch wurde auf 90 °C eingeregelt. Der Druck im Aminalreaktor lag während der Anfahrphase bei 1,4 bar absolut. Während einer Anfahrzeit von 45 Minuten wurde die Menge an 32 %iger wässriger Formaldehydlösung, die in den Aminalreaktor eingeleitet wurde, stufenlos von 0 t/h auf 4,95 t/h hochgezogen. Anschließend wurde die Reaktionsmischung aus dem Aminalreaktor in einen Phasentrennapparat gefahren, in dem das Reaktionswasser aus der Aminalreaktion abgetrennt wurde. Die verbliebene organische Phase wurde nun in den ersten Umlagerungstank gepumpt, wobei gleichzeitig eine 30 %ige wässrige Salzsäure, entsprechend einem Protonierungsgrad von 10 % (d. h., pro Mol Aminogruppen werden 0,1 Mol HCl zugegeben) über eine Mischdüse in den Einlauf des Aminals in den ersten Umlagerungstank eindosiert wurde.

Die Umlagerungsreaktion fand bei 50 °C bis 150 °C in einer Reaktorkaskade statt (Schritt c)). Nach vollständiger Reaktion wurde das erhaltene Reaktionsgemisch, wie in den allgemeinen Herstellungsbedingungen von MDA beschrieben, aufgearbeitet. Nach 3 Produktionstagen musste der Aminalreaktor außer Betrieb genommen werden, weil im Aminalabscheider Feststoff ausgefallen war, der eine Reinigung erforderlich machte.

### Beispiel 3 (erfindungsgemäß): Abfahren der MDA-Anlage, wobei Anilin nachgefahren wurde.

Die MDA-Anlage wurde, wie in den allgemeinen Bedingungen für die Herstellung von MDA beschrieben, bei einer Last von 17 t/h MDA betrieben. Nach einem technischen Defekt in den Umlagerungsreaktoren wurde im Aminalreaktor Formalin abgestellt und Anilin 1 Stunde später abgestellt. Die Zugabe von Salzsäure wurde zum gleichen Zeitpunkt wie die Anilinzugabe unterbrochen.

Der Aminalreaktor und die Umlagerungsreaktoren wurden entleert. Nach Behebung des Defekts in den Umlagerungsreaktoren wurde der leere Aminalreaktor mit Einsatzanilin (enthaltend 90 Massen-% Anilin) befüllt bis Anilin über das Siphon in den Aminalabscheider überlief. Wenn der Aminalreaktor so gefüllt war, lief das Einsatzanilin mit einer Last von 12,2 t/h in den gerührten Aminalreaktionskessel, was 50 % der Nennlast entsprach.

Nun wurde der Formalinweg freigegeben. Die Reaktion sprang sofort an und das Reaktionsgemisch wurde auf 90 °C eingeregelt. Der Druck im Aminalreaktor lag während der Anfahrphase bei 1,4 bar absolut. Während einer Anfahrzeit von 45 Minuten wurde die Menge an 32 %iger wässriger Formaldehydlösung, die in den Aminalreaktor eingeleitet wurde, stufenlos von 0 t/h auf 4,95 t/h hochgezogen.

Anschließend wurde die Reaktionsmischung aus dem Aminalreaktor in einen Phasentrennapparat gefahren, in dem das Reaktionswasser aus der Aminalreaktion abgetrennt wurde. Die verbliebene organische Phase wurde nun in den ersten Umlagerungstank gepumpt, wobei gleichzeitig eine 30 %ige wässrige Salzsäure, entsprechend einem Protonierungsgrad von 10 % (d. h., pro Mol Aminogruppen werden 0,1 Mol HCl zugegeben) über eine Mischdüse in den Einlauf des Aminals in den ersten Umlagerungstank eindosiert wurde. Die Umlagerungsreaktion fand bei 50 °C bis 150 °C in einer Reaktorkaskade statt (Schritt c)).

Nach vollständiger Reaktion wurde das erhaltene Reaktionsgemisch, wie in den allgemeinen Herstellungsbedingungen von MDA beschrieben, aufgearbeitet.

Bei erfindungsgemäßer Vorgehensweise wurde während der Abfahrphase verhindert, dass es zu Ablagerungen im Aminalkessel, im Aminalkühler und im Aminalabscheider kam, die dann nach dem folgenden Anfahren der Anlage und im sich anschließenden kontinuierlichen Betrieb weiter wachsen können und schon nach kurzer Zeit eine Verblockung im Aminalkessel, im Aminalkühler, im Aminalabscheider und auch ein Festsitzen der Kühlkreislaufpumpe durch Feststoffablagerungen hervorrufen könnten.

Der Aminalkessel konnte bei richtigem Abfahren der Anlage und bei richtiger sich anschließender Inbetriebnahme der Anlage über einen langen Produktionszyklus von mehreren Monaten störungsfrei betrieben werden. Die Entstehung von unerwünschten Nebenprodukten wie unlösliche polymere Amine etc. wurde signifikant reduziert, und ein späteres Verschneiden der Anfahrware mit reinem MDA oder im schlimmsten Fall die Verbrennung der Anfahrware konnte unterbleiben.

## Patentansprüche

1. Verfahren zur Herstellung von Di- und/oder Polyaminen der Diphenylmethanreihe (MDA) durch Umsetzung von Anilin (1) und Formaldehyd (2) bei einem molaren Verhältnis von Anilin (1) zu Formaldehyd (2) im Regelbetrieb von A/F_{Regelbetrieb}, umfassend die Schritte:
Entweder gemäß einer Variante A)
A.I) Reaktion von Anilin (1) und Formaldehyd (2) in einem Reaktor in Abwesenheit eines sauren Katalysators (3) unter Bildung eines Aminals, wobei Anilin (1) mit einem Massenstrom m₁ und Formaldehyd (2) mit einem Massenstrom m₂ in den Reaktor eingetragen werden und anschließende Trennung des erhaltenen Reaktionsgemisches in eine wässrige und eine organische, Aminal enthaltende Phase;
A.II) Reaktion zumindest eines Teils der in Schritt A.I) erhaltenen organischen, Aminal enthaltenden Phase in einem Reaktor mit Säure (3), wobei das Aminal zu Di- und Polyaminen der Diphenylmethanreihe reagiert;
wobei die Schritte A.I) und A.II) kontinuierlich durchgeführt werden und wobei zum Beenden der Herstellung der Di- und Polyamine der Diphenlymethanreihe die folgenden Schritte durchlaufen werden:
A.I.1) Reduktion des Massenstroms m₂ des Formaldehyds (2) in den Reaktor aus Schritt A.I) beginnend bei einem Zeitpunkt t₀, bis m₂ zu einem Zeitpunkt t₁ null beträgt, wobei t₁ > t₀;
A.I.2) Reduktion des Massenstroms m₁ des Anilins in den Reaktor aus Schritt A.I) bis m₁ zu einem Zeitpunkt t₂ null beträgt, wobei t₂ > t₁ und wobei die Zeitspanne t₂ ― t₁ > 0,5 Stunden bis < 30 Stunden beträgt;
A.II.1) Reduktion des Massenstroms m₃ an Säure (3), bis m₃ null beträgt;
und
wobei die Reduktion der Massenströme m₁ und m₂ derart geschieht, dass bis zum Erreichen des Zeitpunkts t₁ das momentane molare Verhältnis von
dem Reaktor aus Schritt A.I) zugegebenem Anilin (1)
zu
dem Reaktor aus Schritt A.I) zugegebenem Formaldehyd (2),
A/F_{mom.}, stets ≥ 2 und ≥ 1,05 · A/F_{Regelbetrieb} beträgt;
oder gemäß einer Variante B)
B.I) Reaktion von Anilin (1) und Säure (3) in einem Reaktor unter Bildung eines Reaktionsgemisches, das das Aniliniumsalz der verwendeten Säure (3) enthält;
B.II) Reaktion zumindest eines Teils des in Schritt B.I) erhaltenen Reaktionsgemisches mit Formaldehyd (2) in einem Reaktor, optional unter Zufuhr weiteren Anilins (1), optional unter Zufuhr weiterer Säure (3), wobei Di- und Polyamine der Diphenylmethanreihe entstehen;
wobei die Schritte B.I) und B.II) kontinuierlich durchgeführt werden und wobei zum Beenden der Herstellung der Di- und Polyamine der Diphenlymethanreihe die folgenden Schritte durchlaufen werden:
B.II.1) Reduktion des Massenstroms m₂ des Formaldehyds (2) in den Reaktor aus Schritt B.II) beginnend bei einem Zeitpunkt t₀, bis m₂ zu einem Zeitpunkt t₁ null beträgt, wobei t₁ > t₀;
B.1) Reduktion des Massenstroms m₁ des Anilins in den Reaktor aus Schritt B.I) sowie ggf. in den Reaktor aus Schritt B.II), bis m₁ zu einem Zeitpunkt t₂ null beträgt, wobei t₂ > t₁ und wobei die Zeitspanne t₂ ― t₁ > 0,5 Stunden bis < 30 Stunden beträgt;
B.I.2) Reduktion des Massenstroms m₃ der Säure (3) in den Reaktor aus Schritt B.I) sowie ggf. in den Reaktor aus Schritt B.II), bis m₃ null beträgt;
und
wobei die Reduktion der Massenströme m₁ und m₂ derart geschieht, dass bis zum Erreichen des Zeitpunkts t₁ das momentane molare Verhältnis von
dem Reaktor aus Schritt B.I) zugegebenem Anilin (1) und, sofern vorhanden, dem Reaktor aus Schritt B.II) zugegebenem Anilin (1)
zu
dem Reaktor aus Schritt B.II) zugegebenem Formaldehyd (2),
A/F_{mom.}, stets ≥ 2 und ≥ 1,05 · A/F_{Regelbetrieb} beträgt.

2. Verfahren gemäß Anspruch 1, wobei weiterhin während und/oder nach Schritt A.I) bzw. B.II) das erhaltene Gemisch aus dem jeweiligen Reaktor ausgetragen wird.

3. Verfahren gemäß Anspruch 1 oder 2, wobei in beiden Varianten die Zeitspanne t₂ ― t₁ > 0,5 Stunden bis < 10 Stunden beträgt.

4. Verfahren gemäß einem oder mehreren der vorhergehenden Ansprüche, wobei in beiden Varianten die Zeitspanne t₂ ― t₁ > 1 Stunde bis < 5 Stunden beträgt.

5. Verfahren gemäß einem oder mehreren der vorhergehenden Ansprüche, wobei in beiden Varianten der Massenstrom m₁ ≥ 1000 kg/Stunde beträgt.

6. Verfahren gemäß einem oder mehreren der vorhergehenden Ansprüche, wobei in beiden Varianten der Massenstrom m₂ ≥ 300 kg/Stunde beträgt.

7. Verfahren gemäß einem oder mehreren der vorhergehenden Ansprüche, wobei in beiden Varianten nach dem Zeitpunkt t₂ zumindest der Reaktor aus Schritt A.I) bzw. der Reaktor aus Schritt B.I) zumindest teilweise mit Anilin befüllt vorliegt.

8. Verfahren gemäß einem oder mehreren der vorhergehenden Ansprüche, wobei in beiden Varianten der Massenstrom m₃ an Säure (3) frühestens beginnend beim Zeitpunkt t₁ reduziert wird.

9. Verfahren gemäß Anspruch 8, wobei in beiden Varianten der vor Durchführung der Schritte A.I.1) bzw. B.II.1) herrschende Massenstrom m₃ an Säure (3) bis zum Zeitpunkt t₂ beibehalten wird.

10. Verfahren gemäß Anspruch 8 oder 9, bei dem zum Zeitpunkt t₂ in beiden Varianten die Zufuhr weiterer Säure (3) eingestellt wird.

## Claims

1. Process for preparing diamines and/or polyamines of the diphenylmethane series (MDA) by reaction of aniline (1) and formaldehyde (2) as a molar ratio of aniline (1) to formaldehyde (2) in normal operation of A/Fₙₒᵣₘₐₗ operation, which comprises the steps:
either, according to a variant A)
A.I) reaction of aniline (1) and formaldehyde (2) in the absence of an acid catalyst (3) in a reactor to form an aminal, where aniline (1) is introduced at a mass flow rate m₁ and formaldehyde (2) is introduced at a mass flow rate m₂ into the reactor and the resulting reaction mixture is subsequently separated into an aqueous phase and an organic, aminal-containing phase;
A.II) reaction of at least part of the organic, aminal-containing phase obtained in step A.I) with acid (3) in a reactor, with the aminal reacting to form diamines and polyamines of the diphenylmethane series;
wherein the steps A.I) and A.II) are performed continuously and wherein the following steps are carried out to end the production of the diamines and polyamines of the diphenylmethane series:
A.I.1) reduction of the mass flow rate m₂ of the formaldehyde (2) into the reactor of step A.I), commencing at a point in time t₀, until m₂ is zero at a point in time t₁, where t₁ > t₀;
A.I.2) reduction of the mass flow rate m₁ of the aniline into the reactor of step A.I) until m₁ is zero at a point in time t₂, where t₂ > t₁ and where the period of time t₂ ― t₁ is > 0.5 hours to < 30 hours;
A.II.1) reduction of the mass flow rate m₃ of acid (3), until m₃ is zero;
and
where the reduction of the mass flow rates m₁ and m₂ occurs in such a way that, up to attainment of the point in time t₁, the instantaneous molar ratio of
aniline (1) introduced into the reactor of step A.I)
to
formaldehyde (2) introduced into the reactor of step A.I),
A/F_{inst.}, is always ≥ 2 and ≥ 1.05 · A/F_{normal operation};
or, according to a variant B),
B.I) reaction of aniline (1) and acid (3) in a reactor to form a reaction mixture containing the anilinium salt of the acid (3) used;
B.II) reaction of at least part of the reaction mixture obtained in step B.I) with formaldehyde (2) in a reactor, optionally with introduction of further aniline (1), optionally with the introduction of further acid (3), forming diamines and polyamines of the diphenylmethane series;
wherein the steps B.I) and B.II) are performed continuously and wherein the following steps are carried out to end the production of the diamines and polyamines of the diphenylmethane series:
B.II.1) reduction of the mass flow rate m₂ of the formaldehyde (2) into the reactor of step B.II) commencing at a point in time t₀, until m₂ is zero at a point in time t₁, where t₁ > t₀;
B.I.1) reduction of the mass flow rate m₁ of the aniline into the reactor of step B.I) and optionally into the reactor of step B.II), until m₁ is zero at a point in time t₂, where t₂ > t₁ and where the period of time t₂ ― t₁ is > 0.5 hours to < 30 hours;
B.I.2) reduction of the mass flow rate m₃ of the acid (3) into the reactor of step B.I) and optionally into the reactor of step B.II), until m₃ is zero;
and
where the reduction of the mass flow rates m₁ and m₂ occurs in such a way that, up to attainment of the point in time t₁, the instantaneous molar ratio of
aniline (1) introduced into the reactor of step B.I) and, if present, aniline (1) introduced into the reactor of step B.II)
to
formaldehyde (2) introduced into the reactor of step B.II),
A/F_{inst.}, is always ≥ 2 and ≥ 1.05 · A/F_{normal operation}.

2. Process according to Claim 1, wherein the mixture obtained is also discharged from the respective reactor during and/or after step A.I) or B.II).

3. Process according to Claim 1 or 2, wherein the period of time t₂ ― t₁ is > 0.5 hours to < 10 hours in both variants.

4. Process according to one or more of the preceding claims, wherein the period of time t₂ ― t₁ is > 1 hour to < 5 hours in both variants.

5. Process according to one or more of the preceding claims, wherein the mass flow rate m₁ is ≥ 1000 kg/hour in both variants.

6. Process according to one or more of the preceding claims, wherein the mass flow rate m₂ is ≥ 300 kg/hour in both variants.

7. Process according to one or more of the preceding claims, wherein, in both variants, at least the reactor of step A.I) or the reactor of step B.I) is at least partly full of aniline after the point in time t₂.

8. Process according to one or more of the preceding claims, wherein the mass flow rate m₃ of acid (3) is reduced no earlier than commencing at the point in time t₁ in both variants.

9. Process according to Claim 8, wherein, in both variants, the mass flow rate m₃ of acid (3) prevailing before steps A.I.1) or B.II.1) are carried out is maintained up to the point in time t₂.

10. Process according to Claim 8 or 9, wherein the introduction of further acid (3) is stopped at the point in time t₂ in both variants.

## Revendications

1. Procédé pour la préparation de diamines et/ou de polyamines de la série du diphénylméthane (MDA) par transformation d'aniline (1) et de formaldéhyde (2) à un rapport molaire d'aniline (1) à formaldéhyde (2) lors d'un fonctionnement régulier A/F_{fonctionnement régulier}, comprenant les étapes :
soit, selon une variante A)
A.I) réaction d'aniline (1) et de formaldéhyde (2) dans un réacteur en l'absence d'un catalyseur acide (3) avec formation d'un aminal, l'aniline (1) étant introduite dans le réacteur à un flux massique m₁ et le formaldéhyde (2) à un flux massique m₂, et séparation consécutive du mélange réactionnel obtenu en une phase aqueuse et une phase organique contenant l'aminal ;
A.II) réaction d'au moins une partie de la phase organique, contenant l'aminal, obtenue dans l'étape A.I) dans un réacteur avec l'acide (3), l'aminal réagissant en diamines et en polyamines de la série du diphénylméthane ;
les étapes A.I) et A.II) étant réalisées en continu et les étapes suivantes étant parcourues pour terminer la préparation des diamines et des polyamines de la série du diphénylméthane :
A.I.1) réduction du flux massique m₂ du formaldéhyde (2) dans le réacteur de l'étape A.I), démarrant à un moment t₀, jusqu'à ce que m₂ à un moment t₁ vaille zéro, où t₁ > t₀ ;
A.I.2) réduction du flux massique m₁ de l'aniline dans un réacteur de l'étape A.I) jusqu'à ce que m₁ vaille zéro à un moment t₂, où t₂ > t₁ et où la durée t₂―t₁ est > 0,5 heure jusqu'à < 30 heures ;
A.II.1) réduction du flux massique m₃ d'acide (3), jusqu'à ce que m₃ vaille zéro ; et
la réduction des flux massiques m₁ et m₂ étant réalisée de manière telle que jusqu'au moment t₁, le rapport molaire momentané de
l'aniline (1) introduite dans le réacteur de l'étape A.I) au
formaldéhyde (2) introduit dans le réacteur de l'étape A.I),
A/F_{mom.}, soit toujours ≥ 2 et ≥ 1,05 * A/F_{fonctionnement régulier} ;
soit, selon une variante B)
B.I) réaction d'aniline (1) et d'acide (3) dans un réacteur avec formation d'un mélange réactionnel qui contient le sel d'anilinium de l'acide utilisé (3) ;
B.II) réaction d'au moins une partie du mélange réactionnel obtenu dans l'étape B.I) avec du formaldéhyde (2) dans un réacteur, éventuellement avec addition d'aniline (1) supplémentaire, éventuellement avec addition d'acide (3) supplémentaire, des diamines et des polyamines de la série du diphénylméthane se formant ;
les étapes B.I) et B.II) étant réalisées en continu et les étapes suivantes étant parcourues pour terminer la préparation des diamines et des polyamines de la série du diphénylméthane :
B.II.1) réduction du flux massique m₂ du formaldéhyde (2) dans le réacteur de l'étape B.II), démarrant à un moment t₀, jusqu'à ce que m₂ à un moment t₁ vaille zéro, où t₁ > t₀ ;
B.I.1) réduction du flux massique m₁ de l'aniline dans le réacteur de l'étape B.I) ainsi que le cas échéant dans le réacteur de l'étape B.II) jusqu'à ce que m₁ vaille zéro à un moment t₂, où t₂ > t₁ et où la durée t₂―t₁ est > 0,5 heure jusqu'à < 30 heures ;
B.I.2) réduction du flux massique m₃ de l'acide (3) dans le réacteur de l'étape B.I) ainsi que le cas échéant dans le réacteur de l'étape B.II) jusqu'à ce que m₃ vaille zéro ; et
la réduction des flux massiques m₁ et m₂ étant réalisée de manière telle que jusqu'au moment t₁, le rapport molaire momentané de
l'aniline (1) introduite dans le réacteur de l'étape B.I) et, le cas échéant, de l'aniline (1) introduite dans le réacteur de l'étape B.II)
au
formaldéhyde (2) introduit dans le réacteur de l'étape B.II) ,
A/F_{mom.}, soit toujours ≥ 2 et ≥ 1,05 * A/F_{fonctionnement régulier}.

2. Procédé selon la revendication 1, le mélange obtenu pendant et/ou après l'étape A.I) ou B.II) étant en outre évacué du réacteur respectif.

3. Procédé selon la revendication 1 ou 2, où, dans les deux variantes, la durée t₂―t₁ est > 0,5 heure jusqu'à < 10 heures.

4. Procédé selon l'une ou plusieurs des revendications précédentes, où, dans les deux variantes, la durée t₂―t₁ est > 1 heure jusqu'à < 5 heures.

5. Procédé selon l'une ou plusieurs des revendications précédentes, où, dans les deux variantes, le flux massique m₁ est ≥ 1000 kg/heure.

6. Procédé selon l'une ou plusieurs des revendications précédentes, où, dans les deux variantes, le flux massique m₂ est ≥ 300 kg/heure.

7. Procédé selon l'une ou plusieurs des revendications précédentes, où, dans les deux variantes, après le moment t₂, au moins le réacteur de l'étape A.I) ou, selon le cas, le réacteur de l'étape B.I) se trouve au moins partiellement rempli d'aniline.

8. Procédé selon l'une ou plusieurs des revendications précédentes, où, dans les deux variantes, le flux massique m₃ d'acide (3) est réduit au plus tôt à partir du moment t₁.

9. Procédé selon la revendication 8, où, dans les deux variantes, le flux massique m₃ d'acide (3) établi avant la réalisation des étapes A.I.1) ou, selon le cas, B.II.1) est maintenu jusqu'au moment t₂.

10. Procédé selon la revendication 8 ou 9, dans lequel, dans les deux variantes, l'alimentation en acide (3) supplémentaire est réglée jusqu'au moment t₂.
